# EUROPEAN PATENT APPLICATION

(11) **EP 0 809 975 A2**
(43) Date of publication of application: **03.12.1997**
(21) Application number: 97107977.7
(22) Date of filing: 16.05.1997
(51) Int. Cl.: A61B 17/80

(54) **An apparatus comprising a plate and a fastener for connecting the plate to a bone portion**

(30) Priority: 31.05.1996 US 657781
(71) Applicant: ACROMED CORPORATION, Cleveland Ohio 44115 (US)
(72) Inventor: Benzel, Edward C., Albuquerque, New Mexico 87122 (US); Yuan, Hansen A., Fayetteville, New York 13066 (US); DiNello, Alex, Pala Alto, California 94303 (US); Wefers, Michael H., South Eucled, Ohio 44121 (US); Smith, Aaron C., Gibsonia, Pennsylvania 15044 (US)
(74) Representative: Rottmann, Maximilian R.

(57) **Abstract**

An apparatus comprises a member connectable with a bone portion and having surface means defining an opening. A fastener is extendable through the opening in the member to connect the member to the bone portion. The fastener has a first portion for engaging the bone portion, a second portion, and a longitudinal axis. The second portion of the fastener is expandable into engagement with the surface means defining the opening to prevent relative movement between the fastener and the member. The fastener includes an expander for expanding the second portion into engagement with the surface means defining the first opening. The fastener is positionable in any one of a plurality of positions relative to the member in which the longitudinal axis of the fastener extends at an angle relative to the axis of the opening.

## Description

### Background of the Invention

### Technical Field

The present invention relates to connecting a member, such as a plate, to a bone portion.

### Description of the Prior Art

A known apparatus comprising a plate and a fastener for connecting the plate to a bone portion is disclosed in U.S. Patent No. 4,484,570. U.S. Patent No. 4,484,570 discloses a screw engageable with a bone portion and having a clamping part with an internal conical surface. An expander engages the conical surface to force parts of the clamping part apart to clamp the screw to the plate. Thus, the screw and plate are locked together, and the plate is secured to the bone portion by the screw.

### Summary of the Invention

The present invention is an apparatus comprising a fastener extendable through an opening in a member to connect the member to a bone portion. The fastener has a first portion for engaging the bone portion, a second portion, and a longitudinal axis. The second portion of the fastener is expandable into clamping engagement with surface means defining the opening in the member to prevent relative movement between the fastener and the member. The fastener includes means for expanding the second portion into engagement with the surface means defining the opening. Also, the fastener is positionable in any one of a plurality of positions in which the longitudinal axis of the fastener extends at an angle relative to the axis of the opening.

### Brief Description of the Drawings

The foregoing and other features of the present invention will become more apparent to one skilled in the art upon reading the following description of the invention with reference to the accompanying drawings, wherein:
Fig. 1 is a view of a plate connected to a bone portion;
Fig. 2 is a sectional view taken generally along the line 2-2 of Fig. 1;
Fig. 3 is an enlarged exploded view of parts of Fig. 2;
Fig. 4 is a plan view of a part shown in Fig. 3;
Fig. 5 is an enlarged view of parts of Fig. 3;
Fig. 6 is an enlarged view, generally similar to Fig. 5, showing another position of the parts of Fig. 3; and
Fig. 7 is an enlarged view, generally similar to Fig. 5, showing yet another position of the parts of Fig. 3.

### Description of the Preferred Embodiment of the Invention

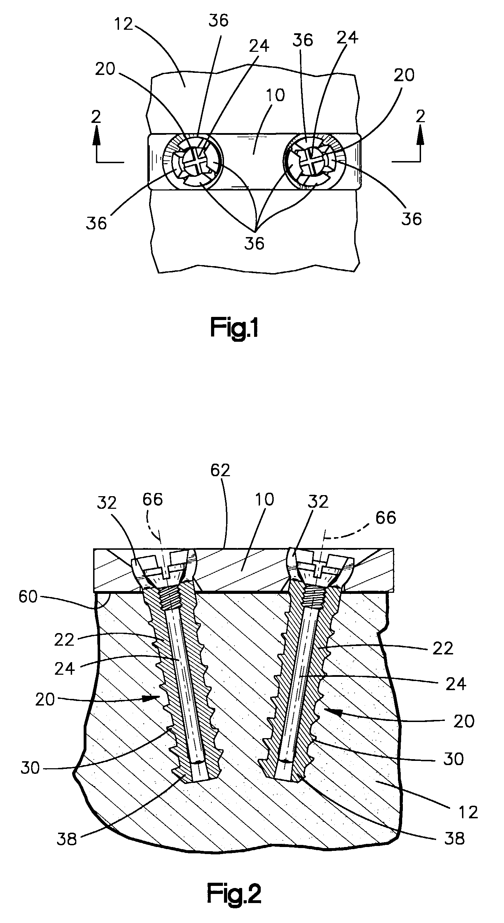

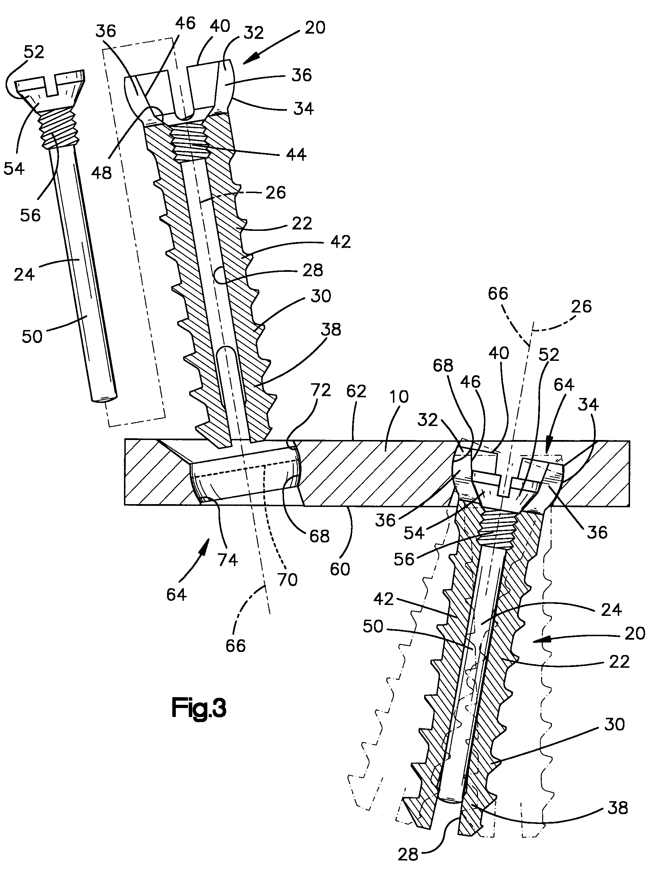

A plate 10 (Fig. 1) is connected to a bone portion 12 by a pair of fasteners 20. Each of the fasteners 20 (Figs. 1 and 2) which secure the plate 10 to the bone portion 12 includes a sleeve 22 and an expander 24 located within the sleeve. Each fastener 20 has a longitudinal central axis 26 (Fig. 3). The sleeve 22 has an axially extending central opening 28 for receiving the expander 24. The sleeve 22 includes a coarse external helical thread convolution 30 for engaging the bone portion 12.

The sleeve 22 (Fig. 3) has a head end portion 32 with a part spherical outer side surface 34. The head end portion 32 of the sleeve 22 is radially and axially slotted to define four segments 36 (Fig. 1) of the head end portion 32. The four segments 36 are movable radially inward and outward relative to each other so that the head end portion 32 is expandable and collapsible. The radially and axially extending slots in the head end portion 32 receive a driving tool for threading the sleeve 22 into the bone portion 12.

An end portion 38 (Fig. 3) of the sleeve 22 opposite from the head end portion 32 is radially and axially slotted to permit radially outward expansion of the end portion 38. The head end portion 32 of the sleeve 22 has a surface 40 facing away from the end portion 38.

The opening 28 of the sleeve 22 has a first diameter located along a central portion 42 (Fig. 3) of the sleeve 22 and a second diameter smaller than the first diameter located adjacent the end portion 38. The sleeve 22 has an internally threaded portion 44 between the central portion 42 and the head end portion 32. The head end portion 32 has a conical shaped surface 46 that tapers from a larger diameter to a smaller diameter adjacent another conical shaped surface 48. The surface 48 interconnects the surface 46 and the threaded portion 44. The surface 48 tapers from a larger diameter adjacent the surface 46 to the smaller diameter of the threaded portion 44.

The expander 24 has a rod-shaped portion 50 for extending into the opening 28. The rod portion 50 has a diameter which is approximately equal to the diameter of the opening 28 in the central portion 42 of the sleeve 22. The rod portion 50 engages the interior of the end portion 38 of the sleeve 22 and causes the end portion 38 to expand and help retain the fastener 20 in the bone portion 12.

The expander 24 has a head end portion 52 with an X-shaped driver slot for receiving a driving tool for rotating the expander relative to the sleeve 22. The head end portion 52 has a tapering surface 54 for engaging the tapering surface 46 of the sleeve 22. The expander 24 includes a threaded portion 56 for threadably engaging the threaded portion 44 of the sleeve 22. The tapering surface 54 of the expander 24 engages the tapering surface 46 of the sleeve 22 to move the four segments 36 radially outward. Therefore, the head end portion 32 of the sleeve 22 expands.

The member or plate 10 (Figs. 2 and 3) is made of a suitable biocompatible material, such as titanium or stainless steel. The plate 10 includes a surface 60 for engaging the bone portion 12 and a surface 62 opposite from the surface 60 for facing away from the bone portion. The plate 10 has a pair of fastener openings 64 for receiving the fasteners 20. The fastener openings 64 have axes 66 extending at an angle to each other.

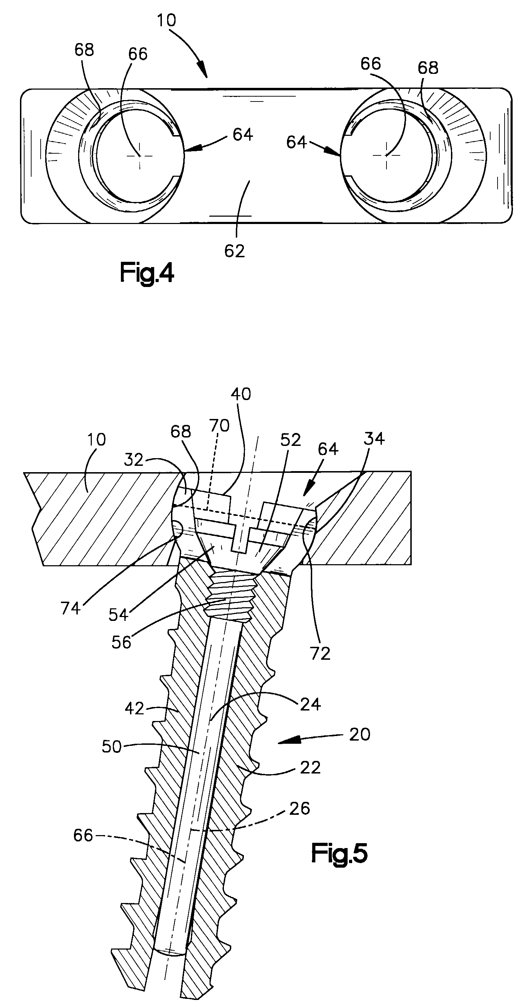

Each one of the fastener openings 64 (Figs. 3 and 4) is partially defined by a part spherical surface 68 centered on the axis 66. The part spherical surface 68 defines a seat or recess in the opening 64 against which the part spherical surface 34 of the sleeve 22 is engageable. The part spherical recess 68 has a major diameter 70. A first portion 72 of the recess 68 is located on one side of the major diameter 70 and adjacent the surface 62 of the plate 10. A second portion 74 of the recess 68 is located on the other side of the major diameter 74 and adjacent the surface 60 of the plate 10.

When the plate 10 is to be connected to a bone portion 12, the plate 10 is placed on the bone portion with the surface 60 engaging the bone portion. The sleeves 22 are threaded into the bone portion 12 through the fastener openings 64 in the plate 10. As the head end portions 32 of the sleeves 22 enter the openings 64 in the plate 10, the segments 36 are compressed radially inward then expand radially outward. The surfaces 40 on the head end portions 32 of the sleeves 22 are located in the first portions 72 of the recesses 68 when the head end portions 32 are received in the recesses. Decause the major diameter 70 of each recess 68 is spaced inward from the outer surface 62 of the plate 10, the expanded fasteners 20 resist movement out of the recesses in the plate.

The expanders 24 are threaded into the sleeves 22 and cause the head end portions 32 of the sleeves to expand into engagement with the openings 64 and prevent relative movement between the plate 10 and the fasteners 20.

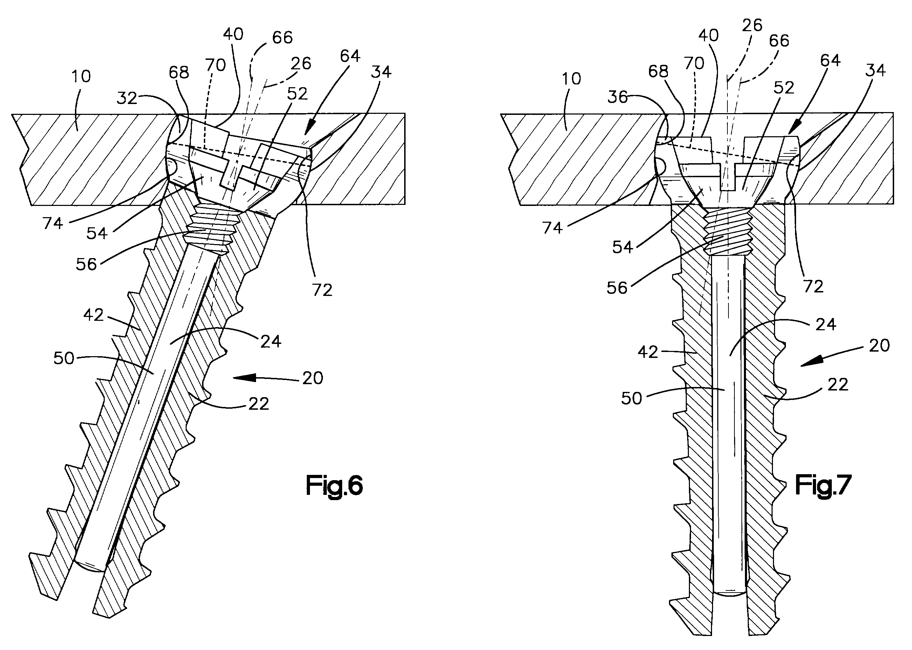

The engagement of the part spherical surfaces 34 of the sleeves 22 with the part spherical surfaces 68 of the openings 64, enables the fasteners 20 to have a plurality of positions in which the axis 26 of each fastener 20 extends at an angle to the axis 66 of its associated opening 64 in any direction. Preferably, the axis 26 of each fastener 20 can be positioned at a maximum of approximately 10° in any direction relative to the axis 66 of its associated opening 64. Three alternative positions are shown in Figs. 5-7.

From the above description of the invention, those skilled in the art will perceive improvements, changes and modifications in the invention. Such improvements, changes and modifications within the skill of the art are intended to be covered by the appended claims.

## Claims

1. An apparatus comprising:
a member connectable with a bone portion, said member having first surface means defining a first opening having a first axis;
a first fastener extendable through said first opening in said member to connect said member to the bone portion, said first fastener having a first portion for engaging the bone portion, a second portion, and a longitudinal axis, said second portion of said first fastener being expandable into engagement with said first surface means defining said first opening in said member to prevent relative movement between said first fastener and said member, said first fastener including means for expanding said second portion of said first fastener into engagement with said first surface means defining said first opening, said first fastener being positionable in any one of a plurality of positions relative to said member in which said longitudinal axis of said first fastener extends at an angle relative to said axis of said first opening.

2. An apparatus as set forth in claim 1 wherein said first surface means defining said first opening in said member defines a partial spherical recess in said member, said second portion of said first fastener being part spherical, said partial spherical recess in said member receiving said part spherical second portion of said first fastener.

3. An apparatus as set forth in claim 2 wherein said partial spherical recess has a major diameter, a first portion located on one side of said major diameter and adjacent a surface of said member for facing away from the bone portion, and a second portion located on another side of said major diameter and adjacent a surface of said member for facing the bone portion.

4. An apparatus as set forth in claim 3 wherein said second portion of said first fastener has a surface facing away from said first portion of said first fastener, said surface on said second portion of said first fastener being located in said first portion of said recess when said second portion of said fastener is received in said recess.

5. An apparatus as set forth in claim 1 wherein said first fastener includes a threaded sleeve for threadably engaging the bone portion and an expander for expanding said second portion of said first fastener, said sleeve having surface means defining an axially extending opening into which said expander is extendable, said surface means defining said axially extending opening in said sleeve having a tapering surface engageable with a tapering surface on said expander to expand said second portion of said first fastener into engagement with said first surface means defining said first opening in said member.

6. An apparatus as set forth in claim 1 further including a second fastener for connecting said member to the bone portion, said member including second surface means defining a second opening having a second axis through which said second fastener is extendable to connect said member to the bone portion, said second fastener having a first portion for engaging the bone portion, a second portion, and a longitudinal axis, said second portion of said second fastener being expandable into engagement with said second surface means defining said second opening in said member to prevent relative movement between said second fastener and said member, said second fastener including means for expanding said second portion of said second fastener into engagement with said second surface means defining said second opening, said second fastener being positionable in any one of a plurality of positions relative to said member in which said longitudinal axis of said second fastener extends at an angle relative to said second axis of said second opening.

7. An apparatus as set forth in claim 6 wherein said second surface means defining said second opening in said member defines a partial spherical recess, said second portion of said second fastener being part spherical, said partial spherical recess in said member receiving said part spherical second portion of said second fastener.

8. An apparatus as set forth in claim 7 wherein said second fastener includes a threaded sleeve for threadably engaging the bone portion and an expander for expanding said second portion of said second fastener, said sleeve having surface means defining an axially extending opening into which said expander is extendable, said surface means defining said axially extending opening in said sleeve having a tapering surface engageable with a tapering surface on said expander to expand said second portion of said second fastener into engagement with said surface means defining said second opening in said member.

9. An apparatus as set forth in claim 1 wherein said first fastener is positionable in any one of a plurality of positions relative to said member in which said longitudinal axis of said first fastener extends at a angle of up to approximately 10° relative to said axis of said first opening.
